# EUROPEAN PATENT APPLICATION

(11) **EP 4 595 986 A1**
(43) Date of publication of application: **06.08.2025**
(21) Application number: 24305176.0
(22) Date of filing: 01.02.2024
(51) Int. Cl.: A61L 2/07, A61L 2/26, B65B 55/02, B65D 51/18, B65D 75/58, B65D 33/16, A61J 1/10, A61J 1/14, A61B 50/00, A61B 50/30

(54) **PROTECTIVE COVER FOR CONTAINER FOR TRANSFER OF STERILIZED COMPONENTS**

(71) Applicant: Becton Dickinson France, 38800 Le Pont-de-Claix (FR)
(72) Inventor: CHASSIN DE KERGOMMEAUX, Liliane, 38000 Grenoble (FR); DORVAL, Bastien, 38410 Saint Martin d'Uriage (FR); LORDEY, Fabien, 38760 Saint Paul De Varces (FR); LAFORET, Hortense, 38000 Grenoble (FR); CHAMBAZ, Sebastien, 38410 Vaulnaveys le Bas (FR); BUCCI, Gregory, 38119 Pierre-Châtel (FR)
(74) Representative: Germain Maureau

(57) **Abstract**

A protective cover for use with a transfer bag includes a receiver having a receiving cavity configured to receive a connection flange and an access door of the transfer bag. A cover is removably connectable to the receiver and is configured to cover at least the receiving cavity of the receiver. A retainer having a first retainer portion on the receiver and a second retainer portion on the cover is configured to receive a portion of the transfer bag. A lock has a first lock portion on the receiver and second lock portion on the cover, and is movable between unlocked and locked positions. In the locked position, the retainer is configured to protect the door assembly of the transfer bag and to protect filling material during filling. The retainer also prevents passage of filling material from the flexible transfer bag into the connection flange and the access door.

## Description

### BACKGROUND

### Field of the Disclosure

The present disclosure relates generally to the packaging and transfer of sterile components used in, e.g., medical devices. More particularly, the present disclosure relates to a protective cover for protecting an access lid or door of a container (i.e., a bag) during filling of sterilized components (e.g., plunger stoppers).

### Description of the Related Art

As is known in the art, transfer or storage devices for delivery of a medicament, drug, or vaccine (such as, e.g., syringes) utilize a plunger stopper in contact with an inside surface of a generally tubular syringe barrel in order to draw a substance into (or expel a substance from) the device by way of a plunger rod.

Currently, many such devices are filled and assembled using automated filling machines. Not only do such machines improve productivity and accuracy, but they also provide for a substantially sterile and aseptic filling environment. The various components of the devices (e.g., plunger stoppers, syringe barrels, etc.) are separately provided within the filling machines to enable at least some level of automated assembly.

Generally, a plurality of plunger stoppers are initially provided in a substantially flexible bag or similar container to be accessed by the assembly machine prior to assembly. The bag generally has an access door that is configured for connecting to a transfer port of an automated assembly machine. The bag is filled with the plunger stoppers using a filling machine. During a filling process, the bag is held in a bag holder with the access door arranged downwards. An open end of the bag opposite the access door is retained in a bag holder and the interior of the bag is filled with the plunger stoppers. After the filling process, the open end of the bag is sealed and the bag and its contents are sterilized via, e.g., gamma irradiation, steam, etc. In this way, the plunger stoppers, are able to be directly transferred from a first sterile environment (i.e., the sterilized bag) to a second sterile environment (i.e., the sterile filling machine).

During the filling process, the bag is often shaken to maximize the fill volume. During such shaking, the bag and/or the access door may contact parts of the filling machine, thereby increasing a potential for damage to the bag and/or the access door. Furthermore, because the plunger stoppers directly contact the inner surface of the access door, the combination of the weight of the plunger stoppers and the shaking of the bag may lead to deformation of the plunger stoppers contacting the access door. In some instances, plunger stoppers may become wedged in the access door.

### SUMMARY

In view of the foregoing, there exists a need for an improved system and method for filling a bag with contents without damaging the bag, the access door, or the contents of the bag during the filling process. Desirably, there is a need for a removable protective cover that addresses the shortcomings of the existing filling process to prevent damage to the bag, the access door, and/or the components during the filling process.

In accordance with some non-limiting embodiments or aspects of the present disclosure, provided is a protective cover configured for use for filling a flexible transfer bag. The protective cover may include a receiver with a first end, a second end, and a receiving cavity between the first end and the second end. The receiving cavity may extend at least through a portion of the receiver and configured to receive a connection flange and an access door of the flexible transfer bag. The protective cover further may include a cover removably connectable to the receiver. The cover may have a first end, a second end, and a cover portion configured to cover at least the receiving cavity of the receiver. The protective cover further may include a retainer having a first retainer portion protruding from the first end of the receiver and a second retainer portion protruding from first end of the cover. The retainer may be configured to receive a portion of the flexible transfer bag in a space between the first retainer portion and the second retainer portion. The protective cover further may include a lock having a first lock portion on the second end of the receiver and a second lock portion on the second end of the cover. The first lock portion and the second lock portion may be movable relative to each other between an unlocked position permitting movement of the cover relative to the receiver and a locked position preventing movement of the cover relative to the receiver. In the locked position, the retainer may be configured to prevent passage of a filling material from the flexible transfer bag into the connection flange and the access door.

In accordance with some non-limiting embodiments or aspects the lock may be gravity-actuated from the unlocked position to the locked position. The first lock portion may include at least one slot, and the second lock portion may include at least one protrusion configured to be slidably received within the at least one slot. The at least one slot may be a pair of slots on opposite sides of the receiving cavity. The at least one slot may include a stepped guiding surface configured to guide the at least one protrusion into the at least one slot.

In accordance with some non-limiting embodiments or aspects, the retainer may extend laterally outward relative to the receiver and the cover. The retainer may include a guide for guiding a movement of the cover relative to the receiver. The guide may include a first guide on a first lateral side of the retainer and a second guide on a second lateral side of the retainer. The guide may include a first guide portion on the retainer and a second guide portion of the cover. The first guide portion may be a channel and the second guide portion may be a pin configured to be slidably received within the channel. The channel may have a curved guiding surface.

In accordance with some non-limiting embodiments or aspects, at least one of the second end of receiver and the second end of the cover may include a bumper. The bumper may be made of an elastomeric material.

In accordance with some non-limiting embodiments or aspects, at least one of the receiver and the cover is made from a rigid plastic material. The rigid plastic material is polyoxymethylene.

In accordance with some non-limiting embodiments or aspects, a filling assembly for filling a flexible transfer bag is provided. The filling assembly may include the flexible bag defining an interior cavity and a connection flange having an opening for accessing the interior cavity; and a protective cover removably connectable to the bag. The protective cover may include a receiver with a first end, a second end, and a receiving cavity between the first end and the second end. The receiving cavity may extend at least through a portion of the receiver and configured to receive the connection flange of the flexible transfer bag. The protective cover further may include a cover removably connectable to the receiver. The cover may have a first end, a second end, and a cover portion configured to cover at least the receiving cavity of the receiver. The protective cover further may include a retainer having a first retainer portion protruding from the first end of the receiver and a second retainer portion protruding from first end of the cover. The retainer may be configured to receive a portion of the flexible transfer bag in a space between the first retainer portion and the second retainer portion. The protective cover further may include a lock having a first lock portion on the second end of the receiver and a second lock portion on the second end of the cover. The first lock portion and the second lock portion may be movable relative to each other between an unlocked position permitting movement of the cover relative to the receiver and a locked position preventing movement of the cover relative to the receiver. In the locked position, the retainer may be configured to prevent passage of a filling material from the flexible transfer bag into the connection flange and the access door.

Various other aspects of the present disclosure are recited in one or more of the following clauses:
Clause 1: A protective cover configured for use for filling a flexible transfer bag, the protective cover comprising: a receiver with a first end, a second end, and a receiving cavity between the first end and the second end, the receiving cavity extending at least through a portion of the receiver and configured to receive a connection flange and an access door of the flexible transfer bag; a cover removably connectable to the receiver, the cover having a first end, a second end, and a cover portion configured to cover at least the receiving cavity of the receiver; a retainer having a first retainer portion protruding from the first end of the receiver and a second retainer portion protruding from the first end of the cover, the retainer configured to receive a portion of the flexible transfer bag in a space between the first retainer portion and the second retainer portion; and a lock having a first lock portion on the second end of the receiver and a second lock portion on the second end of the cover, wherein the first lock portion and the second lock portion are movable relative to each other between an unlocked position permitting movement of the cover relative to the receiver and a locked position preventing movement of the cover relative to the receiver, and wherein, in the locked position, the retainer is configured to prevent passage of a filling material from the flexible transfer bag into the connection flange and the access door.
Clause 2: The protective cover according to clause 1, wherein the lock is gravity-actuated from the unlocked position to the locked position.
Clause 3: The protective cover according to clause 1 or 2, wherein the first lock portion comprises at least one slot, and wherein the second lock portion comprises at least one protrusion configured to be slidably received within the at least one slot.
Clause 4: The protective cover according to clause 3, wherein the at least one slot is a pair of slots on opposite sides of the receiving cavity.
Clause 5: The protective cover according to clause 3 or 4, wherein the at least one slot comprises a stepped guiding surface configured to guide the at least one protrusion into the at least one slot.
Clause 6: The protective cover according to any of clauses 1 to 5, wherein the retainer extends laterally outward relative to the receiver and the cover.
Clause 7: The protective cover according to any of clauses 1 to 6, wherein the retainer comprises a guide for guiding a movement of the cover relative to the receiver.
Clause 8: The protective cover according to clause 7, wherein the guide comprises a first guide on a first lateral side of the retainer and a second guide on a second lateral side of the retainer.
Clause 9: The protective cover according to clause 7 or 8, wherein the guide comprises a first guide portion on the retainer and a second guide portion of the cover.
Clause 10: The protective cover according to clause 9, wherein the first guide portion is a channel and wherein the second guide portion is a pin configured to be slidably received within the channel.
Clause 11: The protective cover according to clause 10, wherein the pin is made of a rigid metal material.
Clause 12: The protective cover according to clause 11, wherein the rigid metal material is stainless steel.
Clause 13: The protective cover according to clause 10, wherein the channel has a curved guiding surface.
Clause 14: The protective cover according to any of clauses 1 to 13, wherein at least one of the second end of receiver and the second end of the cover comprises a bumper.
Clause 15: The protective cover according to clause 14, wherein the bumper is made of an elastomeric material.
Clause 16: The protective cover according to any of clauses 1 to 15, wherein at least one of the receiver and the cover is made from a rigid plastic material.
Clause 17: The protective cover according to clause 16, wherein the rigid plastic material is polyoxymethylene.
Clause 18: The protective cover according to any of clauses 1 to 17, further comprising an indexing element for aligning the cover with a receiver during an initial connection of the cover with the receiver.
Clause 19: The protective cover according to clause 18, wherein the indexing element is made from a rigid plastic material.
Clause 20: The protective cover according to clause 19, wherein the rigid plastic material is polyoxymethylene.
Clause 21: A filling assembly for filling a flexible transfer bag, the filling assembly comprising: the flexible bag defining an interior cavity and a connection flange having an opening for accessing the interior cavity; and a protective cover removably connectable to the bag, the protective cover comprising: a receiver with a first end, a second end, and a receiving cavity between the first end and the second end, the receiving cavity extending at least through a portion of the receiver and configured to receive the connection flange of the flexible transfer bag; a cover removably connectable to the receiver, the cover having a first end, a second end, and a cover portion configured to cover at least the receiving cavity of the receiver; a retainer having a first retainer portion protruding from the first end of the receiver and a second retainer portion protruding from the first end of the cover, the retainer configured to receive a portion of the flexible transfer bag in a space between the first retainer portion and the second retainer portion; and a lock having a first lock portion on the second end of the receiver and a second lock portion on the second end of the cover, wherein the first lock portion and the second lock portion are movable relative to each other between an unlocked position permitting movement of the cover relative to the receiver and a locked position preventing movement of the cover relative to the receiver, and wherein, in the locked position, the retainer is configured to prevent passage of a filling material from the interior cavity of the flexible transfer bag into the connection flange.
Clause 22: The filling assembly according to clause 21, wherein the lock is gravity-actuated from the unlocked position to the locked position.
Clause 23: The filling assembly according to clause 21 or 22, wherein the first lock portion comprises at least one slot, and wherein the second lock portion comprises at least one protrusion configured to be slidably received within the at least one slot.
Clause 24: The filling assembly according to clause 23, wherein the at least one slot is a pair of slots on opposite sides of the receiving cavity.
Clause 25: The filling assembly according to clause 23 or 24, wherein the at least one slot comprises a stepped guiding surface configured to guide the at least one protrusion into the at least one slot.
Clause 26: The filling assembly according to any of clauses 21 to 25, wherein the retainer extends laterally outward relative to the receiver and the cover.
Clause 27: The filling assembly according to any of clauses 21 to 26, wherein the retainer comprises a guide for guiding a movement of the cover relative to the receiver.
Clause 28: The filling assembly according to clause 27, wherein the guide comprises a first guide on a first lateral side of the retainer and a second guide on a second lateral side of the retainer.
Clause 29: The filling assembly according to clause 27 or 28, wherein the guide comprises a first guide portion on the retainer and a second guide portion of the cover.
Clause 30: The filling assembly according to clause 29, wherein the first guide portion is a channel and wherein the second guide portion is a pin configured to be slidably received within the channel.
Clause 31: The filling assembly according to clause 30, wherein the pin is made of a rigid metal material.
Clause 32: The filling assembly according to clause 31, wherein the rigid metal material is stainless steel.
Clause 33: The filling assembly according to clause 30, wherein the channel has a curved guiding surface.
Clause 34: The filling assembly according to any of clauses 21 to 33, wherein at least one of the second end of receiver and the second end of the cover comprises a bumper.
Clause 35: The filling assembly according to clause 34, wherein the bumper is made of an elastomeric material.
Clause 36: The filling assembly according to any of clauses 21 to 35, wherein at least one of the receiver and the cover is made from a rigid plastic material.
Clause 37: The filling assembly according to clause 36, wherein the rigid plastic material is polyoxymethylene.
Clause 38: The filling assembly according to any of clauses 21 to 37, further comprising an indexing element for aligning the cover with a receiver during an initial connection of the cover with the receiver.
Clause 39: The protective cover according to clause 38, wherein the indexing element is made from a rigid plastic material.
Clause 40: The protective cover according to clause 39, wherein the rigid plastic material is polyoxymethylene.

Further details and advantages of the present disclosure will be understood from the following detailed description read in conjunction with the accompanying drawings.

### BRIEF DESCRIPTION OF THE DRAWINGS

FIG. 1 is an exploded schematic view of a transfer container with access door in accordance with an embodiment or aspect of the present disclosure;
FIG. 2 is a perspective view of a protective cover for a transfer container in accordance with an embodiment or aspect of the present disclosure;
FIG. 3 is an exploded view of the protective cover shown in FIG. 2;
FIG. 4 is a top perspective view of the protective cover connected to a transfer container in accordance with an embodiment or aspect of the present disclosure;
FIG. 5 is a top perspective view of the protective cover and transfer container shown in FIG. 4, with a top portion of the protective cover removed;
FIG. 6 is a top perspective view of the protective cover, with the protective cover shown in a closed position;
FIG. 7 is a top perspective view of the protective cover, with the protective cover shown in an open position;
FIGS. 8-10 are detailed perspective views of a joint for connecting a cover to a receiver of the protective cover shown in FIG. 2;
FIG. 11 is a detailed perspective view of an indexing element for aligning a cover with a receiver of the protective cover shown in FIG. 2;
FIG. 12A is a side view of a retainer guide for guiding a movement of a cover relative to a receiver of a protective cover;
FIG. 12B is a perspective view of the retainer guide shown in FIG. 12A;
FIG. 13 is a perspective view of the protective cover installed on a filled transfer container;
FIG. 14 is a detailed view of the protective cover of FIG. 12 with a cover of the protective cover removed; and
FIGS. 15-17 are top perspective views of a protective cover and a transfer container in accordance with an embodiment or aspect of the present disclosure, with a cover of the protective cover shown in various positions relative to a retainer of the protective cover.

### DESCRIPTION OF THE INVENTION

The following description is provided to enable those skilled in the art to make and use the described embodiments or aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

It is noted that any reference to "an embodiment", "one aspect", or "an aspect" means that a particular feature, structure, or characteristic described in connection with the embodiment or aspect is included in at least one embodiment or aspect. Thus, appearances of the phrases "in one embodiment", "in one aspect", or "in an aspect" in various places throughout the specification are not necessarily all referring to the same aspect or embodiment. Furthermore, the particular features, structures or characteristics may be combined in any suitable manner in one or more embodiments or aspects.

One skilled in the art will recognize that the herein described components (e.g., operations), devices, objects, and the discussion accompanying them are used as examples for the sake of conceptual clarity and that various configuration modifications are contemplated. Consequently, as used herein, the specific exemplars set forth and the accompanying discussion are intended to be representative of their more general classes. In general, use of any specific exemplar is intended to be representative of its class, and the non-inclusion of specific components (e.g., operations), devices, and objects should not be taken limiting.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

As used herein, the singular form of "a", "an", and "the" include plural referents unless the context clearly dictates otherwise.

Spatial or directional terms, such as "left", "right", "inner", "outer", "above", "below", and the like, relate to the embodiments or aspects as shown in the drawing figures and are not to be considered as limiting as the embodiments or aspects can assume various alternative orientations.

The terms "first", "second", and the like are not intended to refer to any particular order or chronology, but refer to different conditions, properties, or elements.

The term "at least" is synonymous with "greater than or equal to".

The word "comprising" and "comprises", and the like, does not exclude the presence of elements or steps other than those listed in any claim or the specification as a whole. In the present specification, "comprises" means "includes" and "comprising" means "including".

As used herein, the terms "parallel" or "substantially parallel" mean a relative angle as between two objects (if extended to theoretical intersection), such as elongated objects and including reference lines, that is from 0° to 5°, or from 0° to 3°, or from 0° to 2°, or from 0° to 1°, or from 0° to 0.5°, or from 0° to 0.25°, or from 0° to 0.1°, inclusive of the recited values.

As used herein, the terms "perpendicular", "transverse", "substantially perpendicular", or "substantially transverse" mean a relative angle as between two objects at their real or theoretical intersection is from 85° to 90°, or from 87° to 90°, or from 88° to 90°, or from 89° to 90°, or from 89.5° to 90°, or from 89.75° to 90°, or from 89.9° to 90°, inclusive of the recited values.

If a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

Some aspects may be described using the expression "coupled" and "connected" along with their derivatives. It should be understood that these terms are not intended as synonyms for each other. For example, some aspects may be described using the term "connected" to indicate that two or more elements are in direct physical or electrical contact with each other. In another example, some aspects may be described using the term "coupled" to indicate that two or more elements are in direct physical or electrical contact. The term "coupled," however, also may mean that two or more elements are not in direct contact with each other, but yet still co-operate or interact with each other.

The herein described subject matter sometimes illustrates different components contained within, or connected with, different other components. It is to be understood that such depicted architectures are merely exemplary, and that in fact many other architectures may be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermediate components. Likewise, any two components so associated can also be viewed as being "operably connected," or "operably coupled," to each other to achieve the desired functionality, and any two components capable of being so associated can also be viewed as being "operably couplable," to each other to achieve the desired functionality. Specific examples of operably couplable include but are not limited to physically mateable and/or physically interacting components.

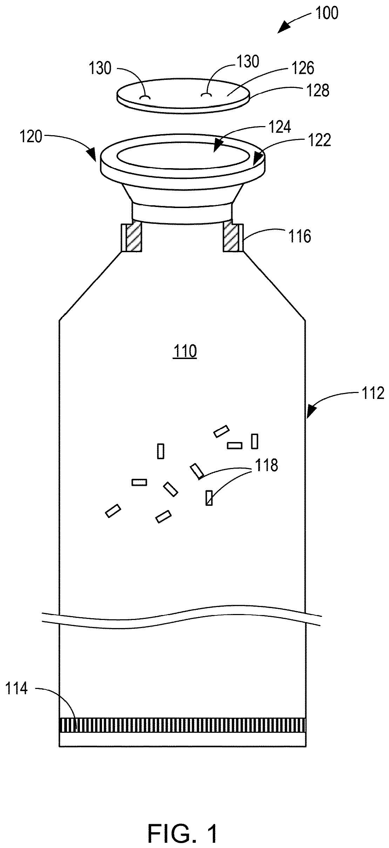

With reference to FIG. 1, a component transfer container 100 (hereinafter referred to as "transfer container 100") is shown in accordance with an embodiment or aspect of the present disclosure. The transfer container 100 is configured for transfer of sterilized components from the transfer container 100 into a sterile chamber of a processing machine through a transfer port. The transfer container 100 includes a flexible bag 112 having a distal end 114 and a proximal end 116 and defining an interior cavity 110. The distal end 114 is closed, while the proximal end 116 is open, preferably in the form of an annular opening. In some embodiments, the distal end 114 may be initially open and is configured to be closed after the interior of the flexible bag 112 is filled with components. In some embodiments or aspects, the flexible bag 112 may taper toward the proximal end 116 in the form of, e.g., a bottleneck. The flexible bag 112 may be formed of any appropriate flexible material suitable for steam sterilization, gamma sterilization, or ethylene oxide sterilization. Examples of such a flexible material may be, e.g., polyethylene (PE), high-density polyethylene (HDPE), thermoplastic elastomer (TPE), polypropylene, polyvinylidene fluoride (PVDF), etc. Additionally, the interior cavity 110 of the flexible bag 112 is sized and configured to hold a plurality of sterilized components 118 therein, such as, e.g., a plurality of plunger stoppers, a plurality of needle covers, a plurality of tip caps, etc.

With continued reference to FIG. 1, transfer container 100 further includes a connection flange 120. The connection flange 120 may be formed of any appropriate rigid material suitable for steam sterilization, gamma sterilization, or ethylene oxide sterilization, such as, e.g., polycarbonate (PC), acrylonitrile butadiene styrene (ABS), polyvinylidene fluoride (PVDF), polybutylene terephthalate (PBT), etc. The connection flange 120 is configured to support the locking forces against a door of a transfer portal of a sterile chamber. In some embodiments or aspects, the connection flange 120 includes a distal end configured for a twist-lock connection to the transfer portal. However, it is to be understood that connection flange 120 may be configured for other forms of connection to the transfer portal, such as, e.g., a magnetic connection.

As shown in FIG. 1, a proximal end of the connection flange 120 includes an annular neck 122. The proximal end 116 of the flexible bag 112 is affixed to the annular neck 122 by way of a rotational seal. Any appropriate method of sealing such as, e.g., heat sealing, adhesive sealing, etc., may be utilized in forming the rotational seal between the proximal end 116 and the annular neck 122. In this way, the contents of flexible bag 112 (i.e., components 118) may be funneled through an opening 124 formed in the connection flange 120 when the transfer container 100 is coupled to a transfer port.

With continued reference to FIG. 1, the transfer container 100 includes an access door 126. The access door 126 is removably connectable to the connection flange 120 to permit access to the interior cavity 110 of the transfer container 100. In some embodiments or aspects, the access door 126 is configured to seal the transfer container 100 to maintain the sterility of the interior cavity 110 after sterilization. The access door 126 may be completely removable from the connection flange 120 to permit unobstructed access into the interior cavity 110. In some embodiments or aspects, the access door 126 may remain connected to the connection flange 120 and may be movable between an open position to permit access into the interior cavity 110, and a closed position to seal the connection flange 120 and prevent access to the interior cavity 110. The access door 126 has a sealing surface 128 configured for a sealed engagement with the connection flange 120. The sealing surface 128 may be an O-ring or a similar sealing structure configured for maintaining the sterility of the interior cavity 110 by preventing ingress of outside contaminants into the interior cavity 110.

The access door 126 may include one or more handling features 130 to facilitate connecting or disconnecting the access door 126 to and from the connection flange 120. For example, the one or more handling features 130 may be one or more handles, pull tabs, or other structures. The access door 126 may be formed of any appropriate rigid material suitable for steam sterilization, gamma sterilization, or ethylene oxide sterilization, such as, e.g., polycarbonate (PC), acrylonitrile butadiene styrene (ABS), polyvinylidene fluoride (PVDF), polybutylene terephthalate (PBT), etc.

With continued reference to FIG. 1, the interior cavity 110 of the flexible bag 112 is filled with components when the flexible bag 112 is in an inverted position such that the proximal end 116 points downwards and an open distal end 114 is configured to receive the components 118 into the interior cavity 110. During the filling operation, the transfer container 100 may be manipulated such that it is lifted and lower repeatedly, such as by shaking in an up and down and/or side-to-side direction, in order to assure even and complete filling of the interior cavity 110 with the components. During such movement, the connection flange 120 and the access door 126 may be damaged. Furthermore, the flexible bag 112 may be folded repeatedly during the filling process, which may compromise the integrity of the sidewall material of the flexible bag 112. Additionally, as the components 118 fill the interior cavity 110 of the flexible bag 112, the components 118 may block the access door 126 and make it difficult to remove.

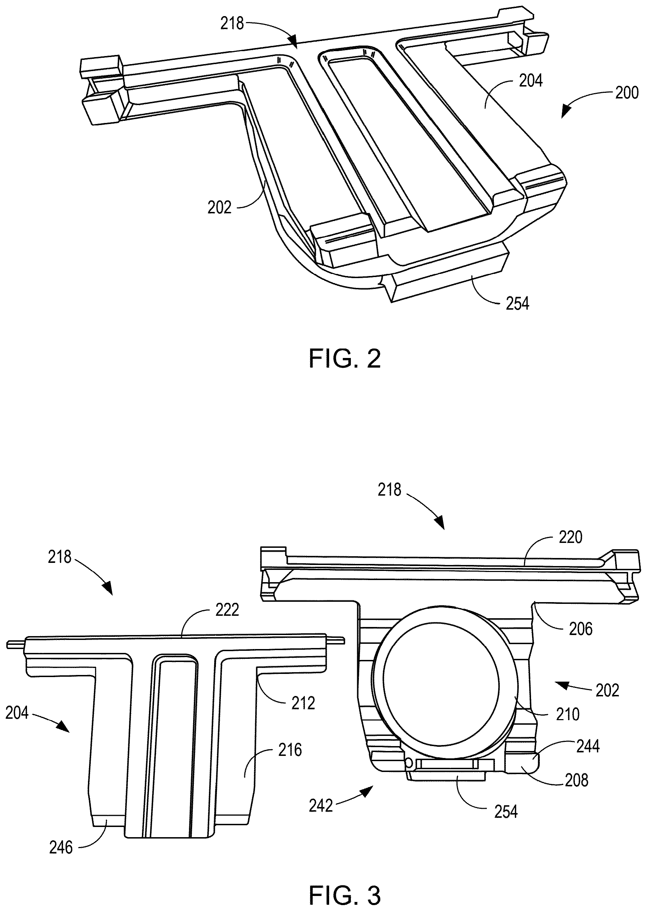

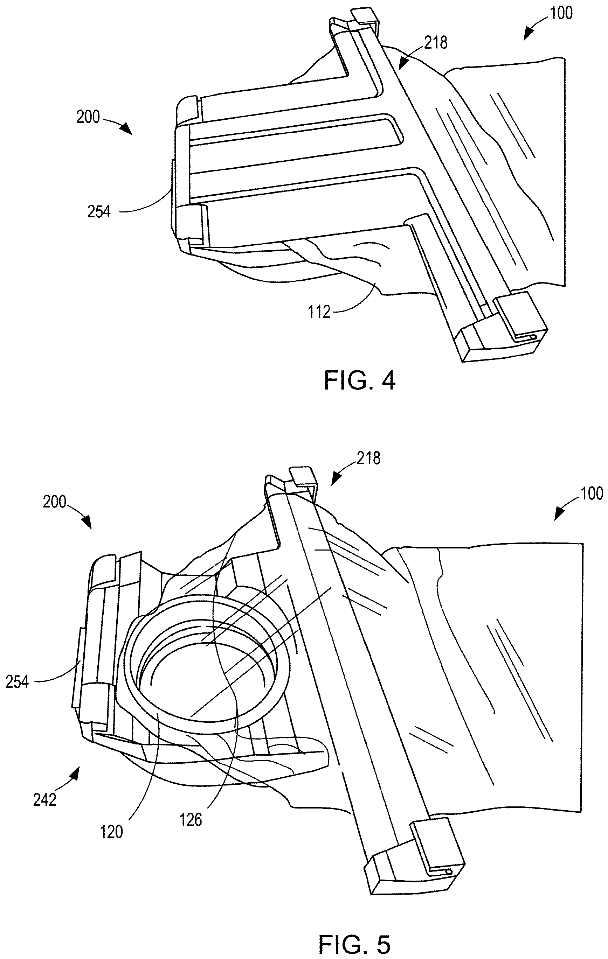

With reference to FIGS. 2-5, the transfer container 100 (shown in FIGS. 4-5) may be provided with a protective cover 200 configured to facilitate filling of the flexible bag 112. Together, the protective cover and the transfer container 100 constitute a filling assembly 300 (shown in FIG. 4). The transfer container 100 and the protective cover 200 are provided as separate components that may be used independently.

The protective cover 200 is configured to protect the connection flange 120 and the access door 126 of the transfer container 100 from damage during manipulation of the transfer container 100 during a filling operation. The protective cover 200 is further configured to prevent damage to the flexible bag 112 and to prevent blockage of the access door 126 with the components 118 as the interior cavity 110 of the flexible bag 112 is filled with the components 118.

The protective cover 200 is configured to be removably connected to the transfer container, such as the connection flange 120, and at least partially enclose the connection flange 120 and the access door 126 to prevent the components 118 from blocking the access door 126. The protective cover 200 is further configured to retain at least a portion of the flexible bag 112 at the proximal end 116 to prevent passage of the components 118 toward the connection flange 120 and the access door 126. The protective cover 200 is further configured to permit filling of the interior cavity 110 of the flexible bag 112 without reducing the capacity of the flexible bag 112.

With reference to FIG. 3, the protective cover 200 has a pair of components that are removably connectable to each other to at least partially enclose the connection flange 120 and the access door 126 of the transfer container 100. In some embodiments or aspects, the protective cover 200 has a receiver 202 and a cover 204 that is removably connectable to the receiver 202. The receiver 202 has a first end 206, a second end 208, and a receiving cavity 210 between the first end 206 and the second end 208. The receiving cavity 210 may extend at least through a portion of the receiver 202 and be configured to receive the connection flange 120 and the access door 126 of the transfer container 110. As shown in FIG. 5, the receiving cavity 210 may be sized to be slightly larger than the connection flange 120 and the access door 126 such that the connection flange 120 and the access door 126 can be easily placed into and removed from the receiving cavity 120.

With continued reference to FIG. 3, the cover 204 may have a first end 212, a second end 214, and a cover portion 216 configured to cover at least the receiving cavity 210 of the receiver 202. When the cover 204 is connected to the receiver 202, such as shown in FIGS. 2 and 5, the cover portion 216 is configured to extend over the receiving cavity 210 to prevent the connection flange 120 and the access door 126 from falling out of the protective cover 200. In accordance with some non-limiting embodiments or aspects, at least one of the receiver 202 and the cover 204 is made from a rigid plastic material. The rigid plastic material may be polyoxymethylene or any other rigid plastic material, such as PC, ABS, TVDF, PBT, etc. In some embodiments or aspects, at least one of the receiver 202 and the cover 204 may be made from metal and/or a combination of rigid plastic material and metal.

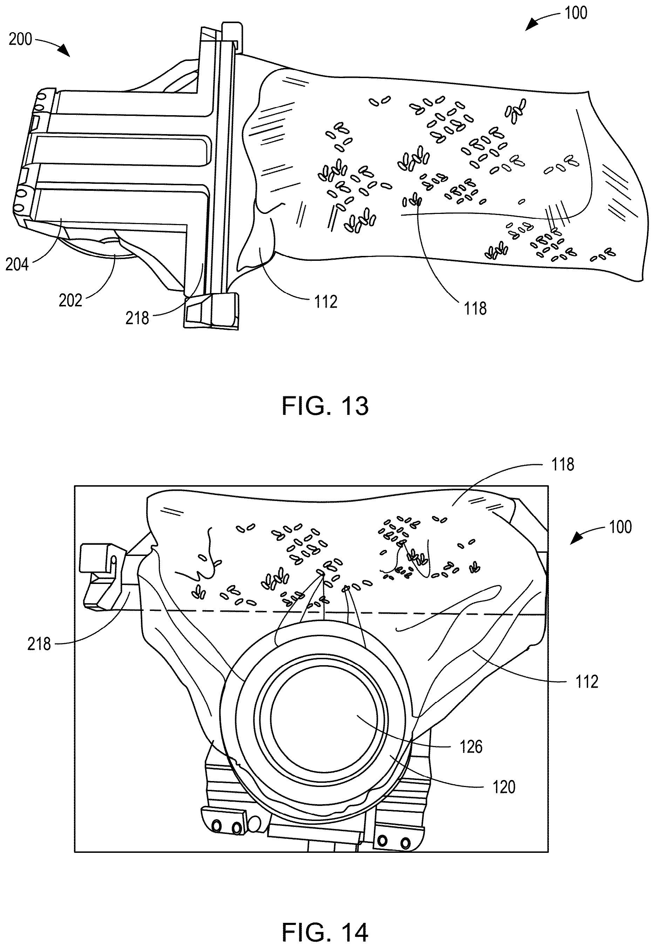

With continued reference to FIG. 3, the protective cover 200 has a retainer 218 extending laterally outward relative to the receiver 202 and the cover 204. The retainer 218 may be configured to retain at least a portion of the flexible bag 112 in a desired positon during a filling operation and prevent a passage of components from the interior cavity 110 of the flexible bag 112 toward the connection flange 120 and the access door 126 (see FIGS. 13-14). The retainer 218 has a first retainer portion 220 protruding from the first end 206 of the receiver 202 and a second retainer portion 222 protruding from first end 212 of the cover 204. The retainer 218 is desirably wider than a width of the flexible bag 112 such that the entire width of the flexible bag 112 can be received in the retainer 218. As shown in FIGS. 4-5, the retainer 218 may be configured to receive a portion of the flexible bag 112 in a space between the first retainer portion 220 and the second retainer portion 222. For example, as shown in FIG. 5, the flexible bag 112 may be folded at the annular neck 122 such that the material of the flexible bag 112 passes through the retainer 218. In some embodiments or aspects, a space between the first retainer portion 220 and the second retainer portion 222 may be wide enough to allow the material of the flexible bag 112 to pass therethrough without allowing the components 118 from the interior cavity 110 of the flexible bag 112 to pass through toward the connection flange 120 and the access door 126.

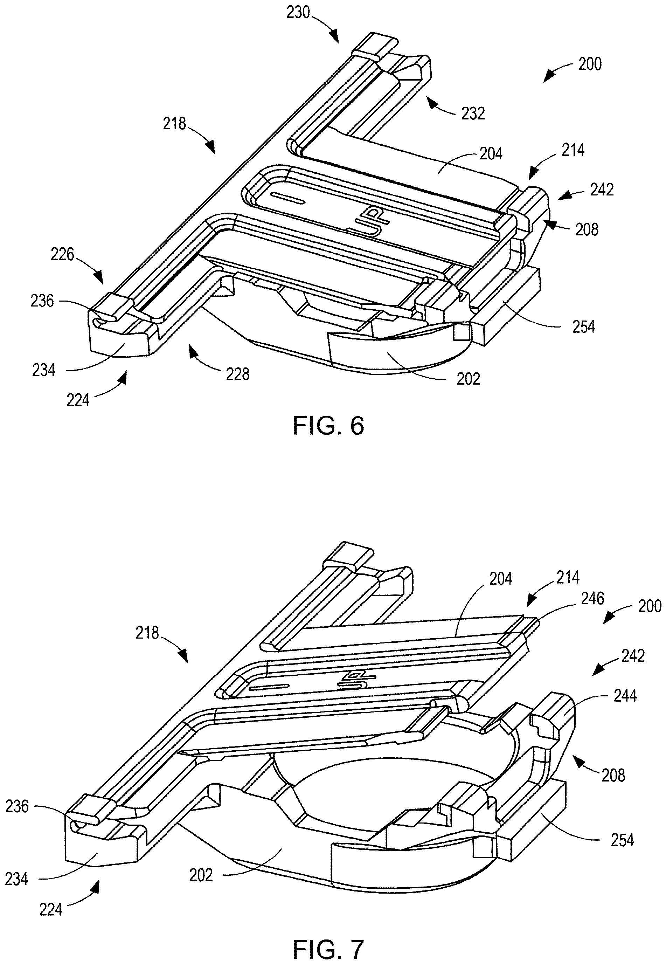

With reference to FIGS. 6-7, the cover 204 is movable relative to the receiver 202 between an open position and a closed position. In the open position, the cover 204 may be removed from the receiver 202 to allow the flexible bag 112 to be inserted into the retainer 218 and the connection flange 120 and the access door 126 to be inserted into the receiving cavity 210 of the receiver 202.

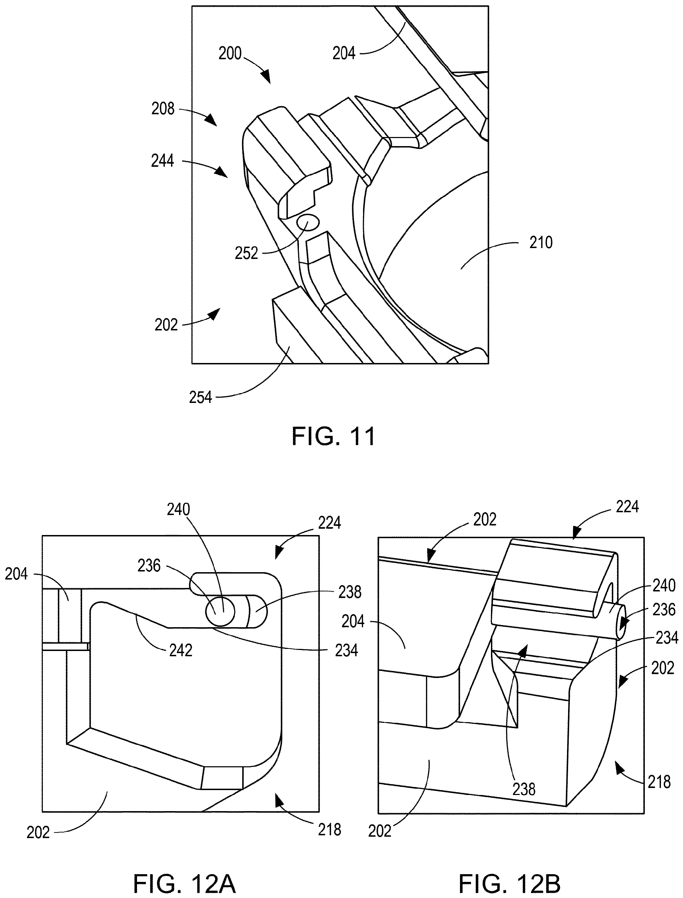

As shown in FIGS. 12A-12B, the retainer 218 has a guide 224 for guiding a movement of the cover 204 relative to the receiver 202. As shown in FIGS. 6-7, the guide 224 may include a first guide 226 on a first lateral side 228 of the retainer 202 and a second guide 230 on a second lateral side 232 of the retainer 202. As shown in FIGS. 12A-12B, the guide 224 has a first guide portion 234 on the retainer 202 and a second guide portion 236 of the cover 204. The first and second guide portions 234, 236 are configured to interact with each other to guide the movement of the cover 204 from the open position to the closed position, and vice versa. The first guide portion 234 may be a channel 238 and the second guide portion 236 may be a pin 240 configured to be slidably received within the channel 238. The pin 240 may be made from a rigid metal material, such as stainless steel. In some embodiments or aspects, the channel 238 may have a curved guiding surface 242 to guide the movement of the pin 240 into the channel 238.

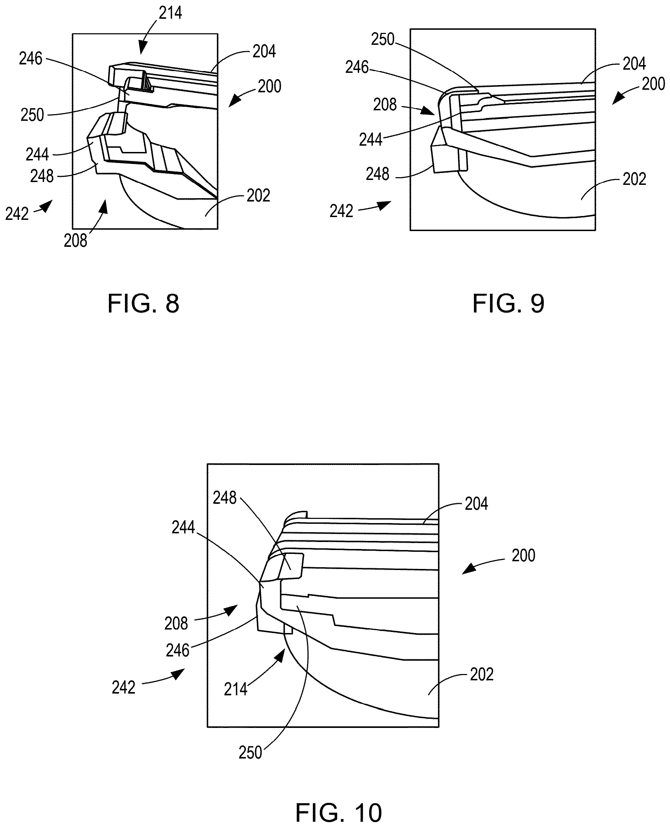

With reference to FIGS. 8-10, the protective cover 200 has a lock 242 configured to lock the cover 204 relative to the receiver 202 and prevent movement of the cover 204 relative to the receiver 202 during the filling operation. In some embodiments or aspects, the lock 242 has a first lock portion 244 on the second end 208 of the receiver 202 and a second lock portion 246 on the second end 214 of the cover 204. The first lock portion 244 and the second lock portion 246 may be movable relative to each other between an unlocked position (FIG. 8) and a locked position (FIG. 10). In the unlocked position, the first lock portion 246 and the second lock portion 246 are configured to permit movement of the cover 204 relative to the receiver 202. In the locked position, the first lock portion 242 and the second lock portion 244 are configured to prevent movement of the cover 204 relative to the receiver 202. Furthermore, in the locked position, the retainer 218 may be configured to prevent passage of a filling material from the flexible bag into the connection flange 120 and the access door 126.

With continued reference to FIGS. 8-10, the first lock portion 244 has at least one slot 248, and the second lock portion 246 has at least one protrusion 250 configured to be slidably received within the at least one slot 248. In some embodiments or aspects, the at least one slot 248 may be a pair of slots 248 on opposite sides of the receiving cavity 210. The at least one slot 248 may have a stepped guiding surface 249 configured to guide the at least one protrusion 250 into the at least one slot 250. With reference to FIG. 11, the protective cover 200 may have an indexing element 252 for aligning the access door 126 with a receiver 202 during an initial connection of the access door 126 with the receiver 202. The access door 126 may have a feature that is configured for alignment with the indexing element 252 during installation of the protective cover 200 on the transfer container 100. In some embodiments, the indexing element 252 has a different color compared to the rest of the components such that the indexing element 252 can be located easily. The indexing element 252 may be made from a rigid plastic material, such as polyoxymethylene.

With reference to FIGS. 6-7, at least one of the second end 208 of receiver 202 and the second end 214 of the cover 204 may include a bumper 254. The bumper 254 may be made of an elastomeric material and may be configured to absorb impacts during a filling operation, such as due to repeated shaking of the transfer container 100. The bumper 254 further may be configured to prevent a direct impact on the receiver 202 and/or the cover 204 against a portion of a filling machine during a filling operation. In some embodiments or aspects, the bumper 254 is removably connectable to the receiver 202 and/or the cover 204 such that the bumper 254 can be replaced without replacing the receiver 202 and/or the cover 204.

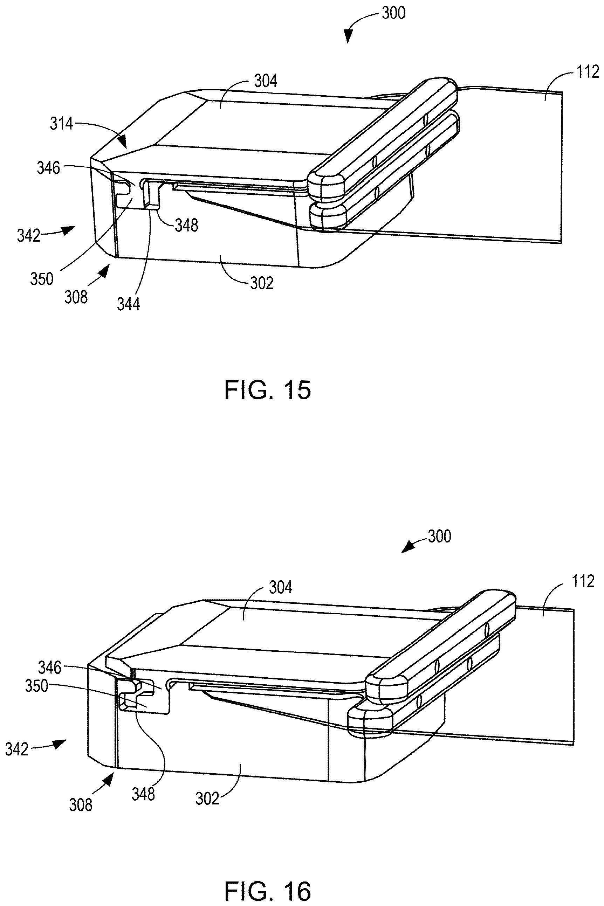

With reference to FIGS. 15-17, a protective cover 300 is shown in accordance with another embodiment or aspect of the present disclosure. The protective cover 300 has substantially similar or identical features as the protective cover 300 described herein with reference to FIGS. 2-14. As such, a detailed discussion of the features of the protective cover 300 will be omitted. Reference numbers used in FIGS. 15-17 to describe the features of the protective cover 300 are the same as reference numbers used in FIGS. 2-14 to describe the features of the protective cover 200, with the exception that the first digit is a "3" instead of a "2" (e.g., protective cover 300 vs. protective cover 200).

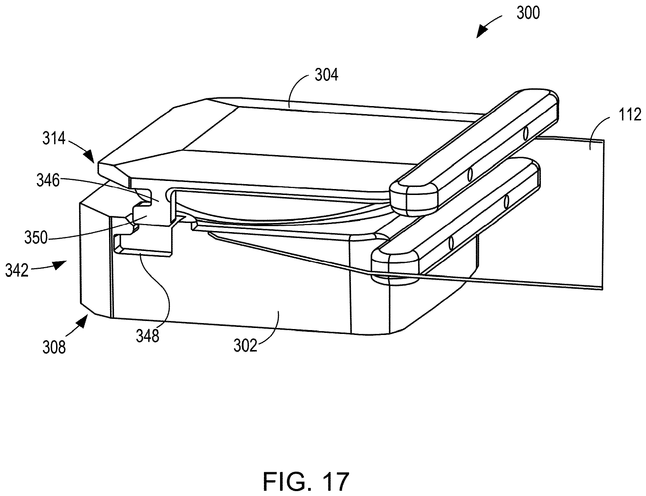

As shown in FIGS. 15-17, the protective cover 300 has a lock 342 configured to lock the cover 304 relative to the receiver 302 and prevent movement of the cover 304 relative to the receiver 302 during the filling operation. In some embodiments or aspects, the lock 342 has a first lock portion 344 on the second end 308 of the receiver 302 and a second lock portion 346 on the second end 314 of the cover 304. The first lock portion 344 and the second lock portion 346 may be movable relative to each other between an unlocked position (FIG. 17) and a locked position (FIG. 15). In the unlocked position, the first lock portion 346 and the second lock portion 346 are configured to permit movement of the cover 304 relative to the receiver 302. In the locked position, the first lock portion 342 and the second lock portion 344 are configured to prevent movement of the cover 204 relative to the receiver 202.

Similar to the embodiment or aspect shown in FIGS. 8-10, the first lock portion 344 in FIGS. 15-17 has at least one slot 348, and the second lock portion 346 has at least one protrusion 350 configured to be slidably received within the at least one slot 348. In some embodiments or aspects, the at least one slot 348 may be L-shaped and be configured to receive a correspondingly-shaped protrusion 350.

Having described the structure of the protective cover 200, 300, a method of using the protective cover 200, 300 will now be described in an exemplary scenario during filling of a flexible bag 112 with components 118. Initially, the connection flange 120 and the access door 126 of the transfer container 100 are inserted into the receiving cavity 210 of the receiver 202. The receiving cavity 210 may be sized to be slightly larger than the connection flange 120 and the access door 126 such that the connection flange 120 and the access door 126 can be easily placed into and removed from the receiving cavity 120. Next, the cover 204 is aligned with the receiver 202 for removably connecting the cover 204 to the receiver 202 to enclose the connection flange 120 and the access door 126. As described herein, by connecting the cover 204 with the receiver 202, at least a portion of the flexible bag 112 is captured in the retainer 218 to prevent passage of the components 118 toward the connection flange 120 and the access door 126. During alignment, the guide 224, such as the first guide 226 and the second guide 230 on the retainer 218, is configured to guide the movement of the cover 204 relative to the receiver 202. Initially, the second guide portion 236, such as the pin 240, engages the curved guiding surface 242 of the channel 238 of the first guide portion 234, which guides the pin 240 toward the closed end of the channel 238. Once the pin 240 is within the channel 238, the second end of the cover 204 may be rotated toward the receiver 202 by pivoting around an axis defined by the pin 240.

When the second end of the cover 204 contacts the second end of the receiver 202, the cover 204 can slide relative to the receiver 202 to engage the lock 242. During engagement of the lock 242, the second lock portion 246 on the cover 204 slides into the first lock portion 244 until the protrusion 250 of the second lock portion 246 is received within the slot 248 of the first lock portion 244. As described herein, the stepped guiding surface 249 is configured to guide the protrusion 250 into place within the slot 248. Such guiding movement may be assisted by gravity, such as when the protective cover 200 is arranged in a vertical orientation wherein the first ends of the receiver 202 and the cover 204 point in an upward direction and the second ends of the receiver 202 and the cover 204 point in a downward direction.

With the lock 242 in a locked position, the open distal end of the flexible bag 112 can be filled with components on a filling machine, with the retainer 218 preventing the passage of the components toward the connection flange 120 and the access door 126. After the interior cavity of the flexible bag is filled with the components, the distal end of the flexible bag can be sealed, and the flexible bag 112 and the protective cover 200 can be removed from the filling machine, and the protective cover 200 can be removed from the connection flange 120 and the access door 126.

While various examples of the present disclosure were provided in the foregoing description, those skilled in the art may make modifications and alterations to these examples without departing from the scope and spirit of the disclosure. Accordingly, the foregoing description is intended to be illustrative rather than restrictive. The disclosure described hereinabove is defined by the appended claims, and all changes to the disclosure that fall within the meaning and the range of equivalency of the claims are to be embraced within their scope.

## Claims

1. A protective cover configured for use for filling a flexible transfer bag, the protective cover comprising:
a receiver with a first end, a second end, and a receiving cavity between the first end and the second end, the receiving cavity extending at least through a portion of the receiver and configured to receive a connection flange and an access door of the flexible transfer bag;
a cover removably connectable to the receiver, the cover having a first end, a second end, and a cover portion configured to cover at least the receiving cavity of the receiver;
a retainer having a first retainer portion protruding from the first end of the receiver and a second retainer portion protruding from the first end of the cover, the retainer configured to receive a portion of the flexible transfer bag in a space between the first retainer portion and the second retainer portion; and
a lock having a first lock portion on the second end of the receiver and a second lock portion on the second end of the cover,
wherein the first lock portion and the second lock portion are movable relative to each other between an unlocked position permitting movement of the cover relative to the receiver and a locked position preventing movement of the cover relative to the receiver, and
wherein, in the locked position, the retainer is configured to prevent passage of a filling material from the flexible transfer bag into the connection flange and the access door.

2. The protective cover according to claim 1, wherein the lock is gravity-actuated from the unlocked position to the locked position.

3. The protective cover according to claim 1 or 2, wherein the first lock portion comprises at least one slot, and wherein the second lock portion comprises at least one protrusion configured to be slidably received within the at least one slot.

4. The protective cover according to claim 3, wherein the at least one slot is a pair of slots on opposite sides of the receiving cavity.

5. The protective cover according to claim 3 or 4, wherein the at least one slot comprises a stepped guiding surface configured to guide the at least one protrusion into the at least one slot.

6. The protective cover according to any of claims 1 to 5, wherein the retainer extends laterally outward relative to the receiver and the cover.

7. The protective cover according to any of claims 1 to 6, wherein the retainer comprises a guide for guiding a movement of the cover relative to the receiver.

8. The protective cover according to claim 7, wherein the guide comprises a first guide on a first lateral side of the retainer and a second guide on a second lateral side of the retainer.

9. The protective cover according to claim 7 or 8, wherein the guide comprises a first guide portion on the retainer and a second guide portion of the cover.

10. The protective cover according to claim 9, wherein the first guide portion is a channel and wherein the second guide portion is a pin configured to be slidably received within the channel.

11. The protective cover according to claim 10, wherein the pin is made of a rigid metal material.

12. The protective cover according to claim 10 or 11, wherein the channel has a curved guiding surface.

13. The protective cover according to any of claims 1 to 12, wherein at least one of the second end of receiver and the second end of the cover comprises a bumper.

14. The protective cover according to claim 13, wherein the bumper is made of an elastomeric material.

15. The protective cover according to any of claims 1 to 14, wherein at least one of the receiver and the cover is made from a rigid plastic material.

16. The protective cover according to any of claims 1 to 15, further comprising an indexing element for aligning the cover with a receiver during an initial connection of the cover with the receiver.

17. The protective cover according to claim 16, wherein the indexing element is made from a rigid plastic material.

18. A filling assembly for filling a flexible transfer bag, the filling assembly comprising:
the flexible bag defining an interior cavity and a connection flange having an opening for accessing the interior cavity; and
a protective cover removably connectable to the bag, the protective cover being as claimed in any of the preceding claims.
